(19)

Europäisches
Patentamt
European
Patent Office
Office européen
des brevets

(11) **EP 2 932 883 A1**

(12) **EUROPEAN PATENT APPLICATION**
published in accordance with Art. 153(4) EPC

(43) Date of publication:
**21.10.2015 Bulletin 2015/43**

(21) Application number: **13863638.6**

(22) Date of filing: **19.11.2013**

(51) Int Cl.:
**A61B 1/00** (2006.01)          **G02B 23/24** (2006.01)

(86) International application number:
**PCT/JP2013/081099**

(87) International publication number:
**WO 2014/091885 (19.06.2014 Gazette 2014/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **11.12.2012 JP 2012270161**

(71) Applicant: **Olympus Corporation
Shibuya-ku
Tokyo 151-0072 (JP)**

(72) Inventors:
• **ITO, Takeshi
Hachioji-shi
Tokyo 192-8512 (JP)**
• **HANE, Jun
Hachioji-shi
Tokyo 192-8512 (JP)**
• **FUJITA, Hiromasa
Hachioji-shi
Tokyo 192-8512 (JP)**
• **TOJO, Ryo
Hachioji-shi
Tokyo 192-8512 (JP)**

(74) Representative: **Winter, Brandl, Fürniss, Hübner,
Röss, Kaiser, Polte - Partnerschaft mbB
Patent- und Rechtsanwaltskanzlei
Bavariaring 10
80336 München (DE)**

(54) **INSERTION-ASSIST INFORMATION DETECTION SYSTEM FOR ENDOSCOPIC DEVICE, AND ENDOSCOPIC DEVICE**

(57)    An insertion assist information detection system (1) includes an endoscope apparatus which includes an insertion portion (12) to be inserted into a tube, a grasp portion (11) which is grasped by an operator, and a movable portion (13) which mechanically connects the insertion portion (12) and the grasp portion (11) to relatively move the insertion portion (12) and the grasp portion (11); and a movement amount detection sensor (50) which detects a relative movement amount of the insertion portion (12) and the grasp portion (11) in the movable portion.

FIG. 1

**Description**

Technical Field

The present invention relates to an insertion assist information detection system for an endoscope apparatus and an endoscope apparatus comprising the same.

Background Art

Heretofore, when a living body is observed or treated by use of an endoscope apparatus, it may be difficult to finely operate the endoscope apparatus depending on the position of an affected part. For example, it is difficult to perform a bending operation depending on the position of an affected part even in the case of an endoscope apparatus called a flexible endoscope capable of bending an insertion portion.

An endoscope apparatus suggested in Jpn. Pat. Appln. KOKAI Publication No. 2005-254002 has an insertion portion, an operation portion, and an insertion portion rotating portion. The insertion portion is a part of the endoscope apparatus to be inserted into an observation target. The operation portion is operated by an operator to issue an operational instruction for the bending state of the insertion portion. The bending state of the insertion portion changes in response to the operational instruction. The insertion portion rotating portion rotates the insertion portion around the longitudinal axis of the insertion portion. The insertion portion can be rotated by the insertion portion rotating portion independently of the operation by the operation portion. According to the endoscope apparatus suggested in Jpn. Pat. Appln. KOKAI Publication No. 2005-254002, work can be done after the insertion portion has been rotated in a direction in which an observation or a treatment can be easily conducted. Thus, a fine treatment can be conducted, and the workability of the operation can be improved.

Disclosure of Invention

In the endoscope apparatus according to Jpn. Pat. Appln. KOKAI Publication No. 2005-254002, the insertion portion is driven independently of the operation portion, thus it may become difficult to recognize the relation between the operation direction of the operation portion and the bending state of the insertion portion in the observation target. In this case, for example, when the inside of a living body is observed or treated, it may be impossible to determine the direction in which the insertion portion should be bended so that the insertion portion will approach an affected part. It may also be impossible to determine the direction in which the insertion portion is viewing at present.

The present invention has been made to solve the above problems, and an object of the invention is to provide an insertion assist information detection system of an endoscope apparatus and an endoscope apparatus using the same which allow a layout relation between an operation portion and an insertion portion to be detected even if the endoscope apparatus can drive the insertion portion independently of the operation portion.

An insertion assist information detection system for an endoscope apparatus according to a first aspect of the invention comprises:

the endoscope apparatus comprising:

an insertion portion to be inserted into a tube,
a grasp portion which is grasped by an operator, and
a movable portion which mechanically connects the insertion portion and the grasp portion to relatively move the insertion portion and the grasp portion; and
a movement amount detection sensor which detects a relative movement amount of the insertion portion and the grasp portion in the movable portion.

An endoscope apparatus according to a second aspect of the invention comprises:

an insertion portion to be inserted into a tube;
a grasp portion which is grasped by an operator;
a movable portion which mechanically connects the insertion portion and the grasp portion to relatively move the insertion portion and the grasp portion; and
a movement amount detection sensor which detects a relative movement amount of the insertion portion and the grasp portion in the movable portion.

Brief Description of Drawings

FIG. 1 is a diagram showing the configuration of an insertion assist information detection system of an endoscope apparatus according to a first embodiment of the present invention;
FIG. 2 is a diagram showing an example of an insertion portion having a configuration in which the direction of the distal end is in alignment with an observation direction;
FIG. 3 is a diagram showing an example of an insertion portion having a configuration in which the direction of the distal end is different from the observation direction;
FIG. 4 is a diagram showing a configuration in the vicinity of a movable portion;
FIG. 5 is a diagram showing a configuration inside an assist information unit;
FIG. 6 is a diagram showing a configuration example of an insertion amount detection sensor;
FIG. 7 is a flowchart showing the flowchart of processing in the insertion assist information detection system of the endoscope apparatus according to the first embodiment of the present invention;
FIG. 8 is a diagram showing an example of a configuration in which the movable portion translates the insertion portion relative to a grasp portion;
FIG. 9 is a diagram showing the configuration of an insertion assist information detection system of an endoscope apparatus according to a second embodiment of the present invention; and
FIG. 10 is a diagram showing the configuration of an insertion assist information detection system of an endoscope apparatus according to a third embodiment of the present invention.

Description of Embodiments

Hereinafter, embodiments of the present invention will be described with reference to the drawings. The embodiments described below suggest an endoscope apparatus which has a movable portion to mechanically connect a grasp portion and an insertion portion to relatively move the grasp portion and the insertion portion and which is provided with a sensor capable of detecting a relative movement amount as insertion assist information, and also suggest an insertion assist information detection system of such an endoscope apparatus. At the same time, the embodiments described below suggest an endoscope apparatus and an insertion assist information detection system of such an endoscope apparatus wherein sensors are disposed in an insertion portion and an observation target, and detection data of these sensors are properly combined to calculate the state and direction of the insertion portion as insertion assist information.

<First Embodiment>

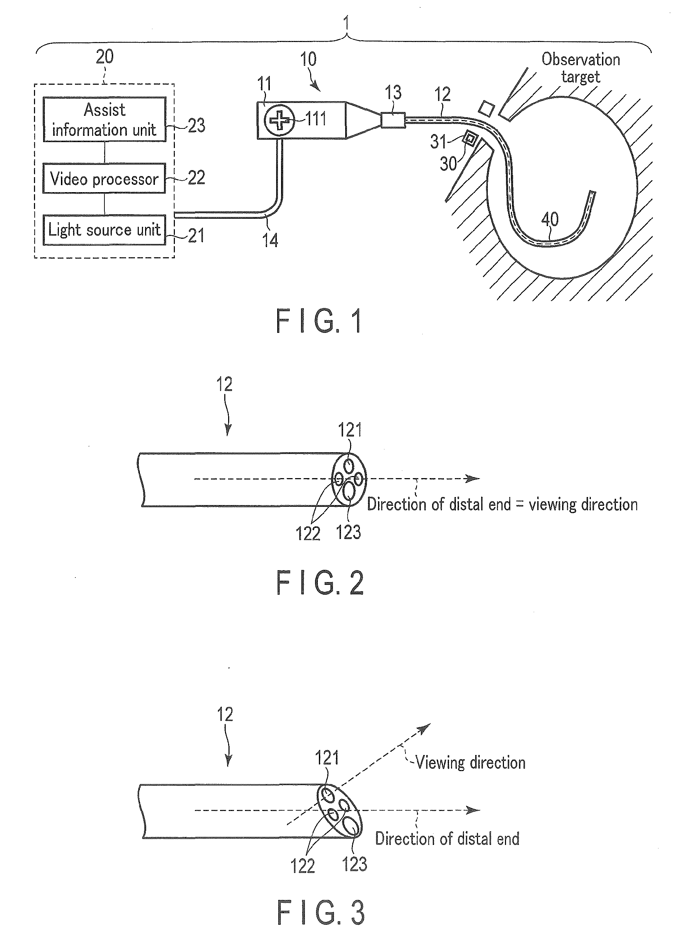

FIG. 1 is a diagram showing the configuration of an insertion assist information detection system of an endoscope apparatus according to the first embodiment of the present invention. As shown in FIG. 1, an insertion assist information detection system 1 according to the present embodiment includes a scope 10, a main body 20, and an insertion amount detection sensor 30. Hereinafter, elements of the insertion assist information detection system 1 are described in the order of the scope 10, the main body 20, and the insertion amount detection sensor 30.

(Scope)

The scope 10 has a grasp portion 11, an insertion portion 12, a movable portion 13, and a main body side cable 14.
The grasp portion 11 is a part of the scope 10 which is configured so that an operator can move while grasping the grasp portion 11 with one hand. The grasp portion 11 is provided with an operation handle 111. The operation handle 111 is operated by the operator and thereby issues an instruction to adjust the bending state of the insertion portion 12.
The insertion portion 12 is a part of the scope 10 which is inserted into a tube such as the internal space of an observation target, and has unshown operation wires provided therein. The operation wires are attached to the operation handle 111. When the operation handle 111 rotates, one of the operation wires is wound up and the other is sent out in response to the rotation. As a result, an unshown bending portion provided in the insertion portion 12 is bent.
As shown in FIG. 2, various members and devices suited to the use of the scope 10, such as an objective lens 121, an illumination portion 122, and a forceps channel 123, are attached to the distal end of the insertion portion 12. Here, the objective lens 121 is a lens optically connected to an image sensor provided inside the insertion portion 12. Light which has entered via the objective lens 121 is received by the image sensor, and converted to an image signal as an electric signal. By transmitting the image signal to the main body 20, it is possible to display, in the main body 20, an image in the direction in which the scope 10 is viewing. The illumination portion 122 is an illumination light emitting portion to emit, as illumination light, light which has been emitted from a light source unit 21 disposed in the insertion portion 12 and guided to the distal end of the insertion portion 12 via an unshown light guide disposed inside the main body side cable 14, the grasp portion 11, and the insertion portion 12. The forceps channel 123 is a bore through which various treatment instruments are inserted. By inserting the various treatment instruments into the forceps channel 123, it is possible to perform various operations and treatments with the scope 10.

Here, the direction in which the objective lens 121 of the scope 10 shown in FIG. 2 is facing is the same as the direction of the axis of the distal end of the insertion portion 12. That is, the direction of the distal end of the insertion portion 12 is equal to the direction in which the objective lens 121 is facing, that is viewing.

On the other hand, as shown in FIG. 3, the scope 10 having a configuration in which the viewing direction of the objective lens 121 is different from the direction of the distal end of the insertion portion 12 may be used. Such a scope 10 can be used for the purpose of observing the side surface of a narrow tube or the wall surface of a space expanding in the far part of a narrow passage.

The insertion portion 12 also has a structure which is pressed against the internal space of the observation target and which is deformable and bendable in accordance with the wall surface thereof, in addition of a bending structure by using the above-mentioned operation wire. According to this structure, the insertion portion 12 can move into the observation target having various introduction passages while being pressed against the inner wall of the observation target.

Furthermore, a bending state detection sensor 40 is mounted inside the insertion portion 12, as shown in FIG. 1. The bending state detection sensor 40 is a sensor which detects the bending state of substantially the whole insertion portion 12, and comprises, for example, an optical fiber sensor. An exemplary optical fiber sensor is disposed substantially over the whole insertion portion 12 so that detection points are dispersed in the longitudinal direction of the insertion portion in such a manner as to be able to detect the shape of the whole insertion portion 12. The configuration and principle of the optical fiber sensor will be described later.

The movable portion 13 is provided between the grasp portion 11 and the insertion portion 12, and mechanically connects the grasp portion 11 and the insertion portion 12 to relatively move the grasp portion 11 and the insertion portion 12. The main body side cable 14 includes one or more cables to electrically and optically connect the scope 10 and the main body 20.

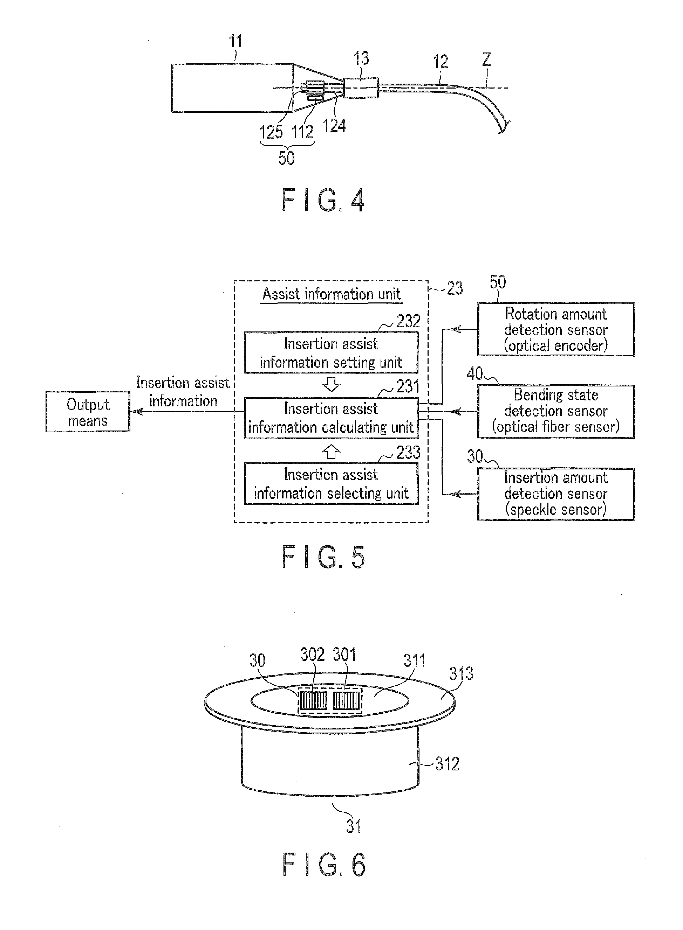

Here, the structure of the movable portion 13 is further described with reference to FIG. 4. FIG. 4 is a diagram showing a configuration in the vicinity of the movable portion 13. As shown in FIG. 4, the insertion portion 12 in the vicinity of the grasp portion 11 has a substantially circular cylindrical shape having a space to pass wiring lines to the image sensor and the like. The movable portion 13 according to the present embodiment is a rotationally movable portion to rotate the insertion portion 12 around a rotation axis Z as the central line of the substantially circular cylindrical shape indicated by a chain line in FIG. 4 which is a direction substantially corresponding to the longitudinal direction of the insertion portion 12. Here, the rotation axis Z is located inside the insertion portion 12 as shown in FIG. 4. The insertion portion 12 has a scope extension 124 which extends to the opposite side across the movable portion 13. Here, the shape of the insertion portion 12 in the vicinity of the grasp portion 11 does not always need to be circular cylindrical. For example, another circular cylindrical member may intervene between the insertion portion 12 and the movable portion 13.

The scope extension 124 has a circular cylindrical shape having a central axis which is the same as the rotation axis of the movable portion 13, and extends to the internal space of the grasp portion 11. The scope extension 124 is configured to rotationally move together with the insertion portion 12 in response to the rotation of the insertion portion 12. That is, in the present embodiment, the insertion portion 12 is configured to be rotationally movable around the rotation axis Z shown in FIG. 4 when the movable portion 13 is rotated. Various mechanical connection structures used in general axial rotation can be used for the rotatable connection structure in the movable portion 13. For example, it is possible to use a configuration that combines a bearing and a circular cylindrical member, or a configuration that combines multiple stages of circular cylinders. A toothed gear or screw threaded configuration may also be used. The movable portion 13 may be designed to be lubricated with oil or the like for smooth movability or to be held with a predetermined strength. The movable portion 13 shown in FIG. 1 doubles as a rotational operation handle which is manually operated when moved by the operator, and the movable portion 13 is fixed to the insertion portion 12 and is configured to be movable relative to the grasp portion 11.

A reflective optical scale 125 in which high reflective portions and low reflective portions are periodically arranged is attached to the outer surface of the scope extension 124 having a circular cylindrical shape. As shown in FIG. 4, an optical sensor head 112 which can be used in combination with the optical scale 125 is attached at a location so as to face the optical scale 125. A rotation amount detection sensor 50, which is a movement amount detection sensor, is configured by the combination of the optical scale 125 and the optical sensor head 112. In the combination according to the present embodiment, the rotation amount detection sensor 50 is a rotary type optical encoder. Various encoders according to existing techniques can be used for the optical encoder. A small encoder is preferable to avoid the size increase of an operation portion. A small encoder that combines an LED with a photodetector array is particularly preferable.

In the example shown in the present embodiment, the optical scale 125 is attached to the side of the insertion portion 12, and the sensor head 112 is attached to the side of the grasp portion 11. As a result, it is possible to avoid the movement of the wiring lines of the sensor head 112 relative to the grasp portion 11. Therefore, the durability of the wiring lines of the sensor head 112 can be improved. Moreover, the structure of the scope extension 124 may remain circularly cylindrical, and a simple structure is possible. In contrast, the sensor head 112 may be provided on the side of the insertion portion 12, and the optical scale 125 may be provided on the side of the grasp portion 11. In this case, the structure is slightly complex, and consideration is needed for the wiring lines, but the basic function is equivalent.

Although the circular cylindrical rotary optical encoder that uses the circular cylindrical optical scale is used in the example shown in the present embodiment, the structure of the rotation amount detection sensor is not limited to the circular cylindrical rotary optical encoder. It is also possible to use an optical encoder that uses a circular plate scale in which a periodic optical pattern is formed on the surface of a disk. In this case, it is possible to produce the circular plate scale at low cost, and it is also possible to increase the diameter of the circular plate to improve the angular resolution at which the encoder can detect. Moreover, it is also possible to use a disk-shaped or circular cylindrical magnetic encoder. By using the magnetic encoder, it is possible to obtain a rotation amount detection sensor that is low-priced and easy to adjust for attachment. Further, it is possible to use an electrostatic encoder and various other encoders.

(Main body)

As shown in FIG. 1, the main body 20 has the light source unit 21, a video processor 22, and an assist information unit 23.

The light source unit 21 has, as a light source, a lamp such as a xenon lamp or a halogen lamp, or a semiconductor light source such as an LED. Illumination light from these light sources is configured to enter the light guide. The light guide is continuously provided through the main body side cable 14, the grasp portion 11, and the insertion portion 12, and is configured so that the illumination light from the light source unit 21 can be emitted from the illumination portion 122 provided at the distal end of the insertion portion 12.

The video processor 22 processes an image signal of the inside of the observation target generated by the image sensor mounted at the distal end of the insertion portion 12 so that the image signal can be displayed on an unshown monitor. This video processor 22 is connected to a signal line. The signal line is continuously provided through the main body side cable 14, the grasp portion 11, and the insertion portion 12, and transmits the image signal from the image sensor to the video processor 22.

The assist information unit 23 processes information from various sensors included in the insertion assist information detection system 1, and outputs the processing results as insertion assist information. The various sensors in the present embodiment are broadly classified into a movement amount detection sensor, a bending state detection sensor, and a layout relation detection sensor. The movement amount detection sensor is a sensor for detecting a relative movement amount of the grasp portion 11 and the insertion portion 12. The rotation amount detection sensor 50 in FIG. 4 corresponds to the movement amount detection sensor. The bending state detection sensor is a sensor for detecting the bending state of the insertion portion 12, and the bending state detection sensor 40 corresponds to the bending state detection sensor in the example in FIG. 1. The layout relation detection sensor is a sensor for detecting a relative layout relation between the observation target and the insertion portion, and the insertion amount detection sensor 30 corresponds to the layout relation detection sensor in the example in FIG. 1. For example, the rotary type optical encoder is used as the rotation amount detection sensor 50. For example, an optical fiber sensor is used as the bending state detection sensor 40. For example, a speckle sensor is used as the insertion amount detection sensor 30.

The assist information unit 23 has an insertion assist information calculating unit 231, an insertion assist information setting unit 232, and an insertion assist information selecting unit 233. The insertion assist information calculating unit 231 has a storage unit to store the information from the various sensors, and uses the information stored in the storage unit to generate insertion assist information. The insertion assist information setting unit 232 stores necessary information needed to convert the information from the various sensors to information that can be calculated in the insertion assist information calculating unit 231, and sets necessary information needed for the insertion assist information calculating unit 231. The necessary information not only includes, for example, information of system of unit regarding the various sensors and layout information but also includes configurational information regarding the scope 10. For example, the configurational information is a rotational amount, in degrees to which one pulse of the rotary type optical encoder as the rotation amount detection sensor 50 corresponds. This rotation amount varies depending on the diameters of the insertion portion 12 and the movable portion 13, thus information corresponding to the kinds of insertion portion 12 and movable portion 13 is stored in the storage unit. The insertion assist information selecting unit 233 determines the insertion assist information needed for the operator from the insertion assist information calculated by the insertion assist information calculating unit 231, and selects the necessary insertion assist information.

The setting by the insertion assist information setting unit 232 and the selection by the insertion assist information selecting unit 233 can be automated. In this case, a program and a data table that conform to a predetermined algorithm can be stored in the storage units of the insertion assist information setting unit 232 and the insertion assist information selecting unit 233. The necessary information may be input and the insertion assist information may be selected from the outside of the assist information unit 23.

The insertion assist information calculated by the insertion assist information calculating unit 231 and selected by the insertion assist information selecting unit 233 is processed into a form that can be output by predetermined output means, and then output to the predetermined output means. Thus, the operator can use the insertion assist information. The output referred to here not only includes visual output that indicates the insertion assist information as image information or character information but also includes audio output such as voice or an alarm sound and tactile information such as vibration. That is, the "output means" in the present embodiment is a generic term covering various existing information transmitting methods that permits information to be transmitted to the operator. In FIG. 1, the video processor 22 is shown as an example of the output means.

In the example shown in the present embodiment, the main body 20 comprises the three units shown in FIG. 1: the light source unit 21, the video processor 22, and the assist information unit 23. However, the main body 20 may also include additional units. For example, the main body 20 can include any units that can be connected to the endoscope apparatus, such as a printer and medical instruments necessary for various procedures and treatments.

Although the assist information unit 23 is shown separately from the video processor 22 and the light source unit 21 in FIG. 1, this is not a limitation. All these units can be formed as one unit, or parts of the functions of the light source unit 21 and the video processor 22 can be combined with the assist information unit 23. Moreover, these units can be integrated with units other than the above-mentioned three units. As the configuration of the assist information unit 23, the insertion assist information calculating unit 231, the insertion assist information setting unit 232, and the insertion assist information selecting unit 233 may be combined into one unit or separately formed or may be each combined with another unit, and can be freely combined in consideration of various circumstances such as the convenience for the operator, ease of designing, and costs.

(Insertion amount detection sensor)

As shown in FIG. 1, the insertion amount detection sensor 30 is a sensor capable of detecting at least one of the length of the insertion portion 12 inserted in the observation target, a rotation amount (insertion torsion amount) of the insertion portion 12 relative to the observation target, and an insertion angle of the insertion portion 12 to the observation target, when the insertion portion 12 is inserted in the observation target. This insertion amount detection sensor 30 is configured to be attachable to the vicinity of an insertion hole of the observation target. Here, in the present embodiment, the speckle sensor is used as the insertion amount detection sensor 30. The speckle sensor is a general optical sensor used for computer mouse input.

The principle of the speckle sensor is briefly described. If light is applied to an object from a coherent light source such as a laser or an LED, reflected light and scattered light from the object interfere with each other, and form a random light-dark pattern on a screen. This random light-dark pattern reflects slight irregularities on the surface of the object or reflecting/nonreflecting patterns. If the same place is irradiated by the same light source under the same conditions, for example, at the same irradiation angle and in the same light amount, the same pattern is formed on a projection surface. Such a pattern is referred to as a speckle pattern. If the object moves relative to the light source, the speckle pattern also moves a distance and in a direction corresponding to the movement direction and movement amount of the object while maintaining its pattern shape. If the movement amount and direction of the speckle pattern are detected by, for example, an image sensor, the movement amount, movement direction, and rotation amount of the object can be detected.

In the present embodiment, a ring-shaped insertion portion adapter 31 is used to attach the insertion amount detection sensor 30 to the insertion opening of the observation target as shown in FIG. 6. FIG. 1 shows the section of the insertion portion adapter 31. In the present embodiment, the insertion amount detection sensor 30 is incorporated in the insertion portion adapter 31. The insertion portion adapter 31 is designed to have the size and shape of an adapter opening 311 adaptive to the opening of the observation target so that the insertion portion adapter 31 is easily attached to the opening of the observation target. FIG. 6 is a view of the insertion portion adapter 31 which is attached to be partly inserted into an opening of a living body such as a mouth or an anus when a living body is the observation target.

The insertion portion adapter 31 shown in FIG. 6 is fixed by, for example, frictional force so that the insertion portion adapter 31 may not rotate or come off when its insertion side surface 312 has come into contact with the opening of the observation target. To prevent the adapter from falling into the observation target, the insertion portion adapter 31 further has a fall prevention portion 313 provided at the end of the adapter circular cylinder which is located outside the observation target when the insertion portion adapter 31 is inserted in the opening of the observation target. The fall prevention portion 313 is a ring-shaped circular plate having an opening in the center, and is designed so that the maximum outside diameter is greater than the opening of the observation target.

Here, in the diagram shown in FIG. 6, the flanged fall prevention portion 313 is provided in the adapter circular cylinder to simplify the explanation. It is preferable that the actual insertion portion adapter 31 is variously elaborated, for example, elliptically shaped or has rounded corners so that the insertion portion adapter 31 may be easily attached to the living body, may not be easily displaced or dropped, may not damage the observation target, and may not be uncomfortable.

The adapter opening 311 of the insertion portion adapter 31 has an open diameter such that the insertion portion 12 can be inserted with an amount of force that may not be a burden to the operator or the observation target. More specifically, the adapter opening 311 has an open diameter larger by a certain amount than the insertion portion 12. However, the adapter opening 311 has an open diameter that is not too large as compared to the insertion portion 12 to prevent erroneous detection by the insertion amount detection sensor 30 as a result of lateral displacement of the insertion portion 12 in the adapter opening 311. Such an adapter opening 311 should be designed in consideration of the use of the scope 10, the environment in which the scope 10 is used, and the accuracy required of the insertion amount detection sensor 30. In the present embodiment, the open diameter of the adapter opening 311 is, for example, a diameter between a diameter slightly larger than the maximum diameter $\varphi$max of the insertion portion 12 and a diameter 3 times of $\varphi$max which is triple the former diameter.

As described above, the insertion amount detection sensor 30 is incorporated in the adapter opening 311. The insertion amount detection sensor 30 has a coherent light source 301 and an image sensor 302. Coherent light emitted from the coherent light source 301 is reflected and scattered by the side surface of the insertion portion 12 inserted in the adapter opening 311, and then forms a two-dimensional speckle pattern on a light receiving surface of the image sensor 302. This speckle pattern moves in response to the insertion direction and insertion amount of the insertion portion 12 in accordance with the above-mentioned principle. Therefore, it is possible to detect the insertion amount, insertion angle, and rotation amount of the insertion portion 12 relative to the observation target by detecting the movement of the speckle pattern.

Here, when the open diameter of the adapter opening 311 is a diameter substantially equal to the maximum diameter $\varphi$max of the insertion portion 12, the insertion angle is limited to the direction that conforms to the adapter opening 311. Therefore, the detection of the insertion angle is unnecessary in this case, and a necessary detection amount can be obtained as the insertion assist information if the insertion amount and the rotation amount can be detected.

Although not shown in FIG. 6, the performance and function of the insertion amount detection sensor 30 can be improved and stabilized if a lens properly designed by a conventional technique is provided at the light input/output terminals of the coherent light source 301 and the image sensor 302.

Various means are possible as the means for supplying electric power to the coherent light source 301 and the image sensor 302 that constitute the insertion amount detection sensor 30 and for taking a detection signal from the image sensor 302. For example, a battery and a wireless signal transmitter are provided in the insertion portion adapter 31, and a signal receiver is provided in, for example, the main body 20, so that the insertion amount detection sensor 30 can have a completely wireless configuration. Moreover, an electric power cable and a signal cable can be wire type cables that are arranged in consideration of the work by the operator. An advantage of the wireless type is that the wiring lines do not lie in the way of the operator and that any place can be used for installation. An advantage of the wire type is that the insertion portion adapter 31 can be reduced in size, weight, and cost. Yet another advantage is that a battery does not run out even after long use.

Although the insertion portion adapter 31 of the type that is directly attached to the opening of the living body is described by way of example according to the present embodiment, this is not a limitation. A seat to directly fix the insertion portion adapter 31 to a bed or the observation target can be provided so that the insertion portion adapter 31 may be disposed in the vicinity of the opening of the observation target. It is also possible to use the insertion portion adapter 31 of a type that is fixed to the seat.

The assist information unit 23 is further described below. As described above, the assist information unit 23 processes the information from the three sensors including the insertion amount detection sensor 30, the bending state detection sensor 40, and the rotation amount detection sensor 50 as the various sensors, and then outputs the insertion assist information. As shown in FIG. 5, the assist information unit 23 has the insertion assist information calculating unit 231, the insertion assist information setting unit 232, and the insertion assist information selecting unit 233. Basic information output from each of the sensors and input to the insertion assist information calculating unit 231 is described below.

The insertion amount detection sensor 30 is, for example, a speckle sensor, is fixed to the vicinity of the opening of the observation target, and detects the length of the insertion portion 12 inserted in the observation target from the opening thereof, a rotation amount of the insertion portion 12, and an insertion angle thereof. As described above, the speckle sensor detects the movement of the speckle pattern, and it is possible to know the actual movement amount and direction of the insertion portion 12 relative to the insertion amount detection sensor 30 by obtaining the relation between the movement amount and direction of the speckle pattern and the relative movement amount and direction of the insertion amount detection sensor 30 and the insertion portion 12. The speckle sensor outputs, as an electric signal, image information regarding the speckle pattern detected by the image sensor 302. An insertion amount detection sensor table, which gives the relation between the movement amount and direction of the speckle pattern and the movement amount and direction of the insertion portion 12, is held in the storage unit of the insertion assist information setting unit 232, and is transmitted at the request of the insertion assist information calculating unit 231.

The insertion assist information calculating unit 231 calculates the length, rotation direction, and insertion direction of the insertion portion 12 inserted in the observation target from the insertion amount detection sensor 30 on the basis of the detection information from the insertion amount detection sensor 30 and the insertion amount detection sensor table from the insertion assist information setting unit 232. In other words, the position and direction of the insertion portion 12 on a coordinate system fixed to the opening of the observation target are calculated.

As described above, the insertion amount detection sensor 30 outputs the image information regarding the speckle pattern as the basic information in the form of an electric signal. The insertion assist information calculating unit 231 calculates insertion assist information by properly combining the output from the insertion amount detection sensor 30, the necessary information from the insertion assist information setting unit 232, and the information from the other sensors.

The bending state detection sensor 40 is a sensor which detects the bending state of the insertion portion 12. In the present embodiment, for example, the optical fiber sensor is used as the bending state detection sensor 40. The optical fiber sensor is a bending sensor in which a detector is provided in a part of the side surface of a long optical fiber and which uses a phenomenon wherein at least one of the amount, wavelength, intensity, and phase of light guided by the optical fiber increases or decreases in response to the bending angle of the optical fiber. As the configuration of the detector, there are known a method of removing the cladding of the optical fiber, and a method of coating the removed part with a light absorbing material. An optical fiber sensor comprising one detector is a bending sensor. An optical fiber sensor having detectors sequentially arranged in the longitudinal direction of the insertion portion 12 is the bending state detection sensor 40 capable of detecting the three-dimensional shape of the insertion portion 12. It is possible to provide detectors in one optical fiber by, for example, means of changing wavelength, and it is also possible to provide multipoint detection by bundling a large number of optical fibers. It is possible to form a thin optical fiber sensor by increasing the number of detection points in one optical fiber. Such an optical fiber sensor having a small diameter is easy to mount in the space of the insertion portion 12. When a large number of optical fibers are bundled into an optical fiber sensor, the independence of the signal at each detection point can be enhanced. Thus, it is possible to enhance the detection accuracy at each detection point and enhance the signal-to-noise ratio.

If about one detector is provided, for example, every 10 cm in the insertion portion 12, the shape of the whole insertion portion 12 can be detected with high reproducibility. If the space between the detectors is smaller than 10 cm, the reproducibility of the shape of the whole insertion portion 12 can be improved. If the space between the detectors is larger than 10 cm, it is possible to reduce costs and simplify the system of the bending state detection sensor 40. The scope 10 can be freely bent in all directions, so that it is necessary to configure an optical fiber sensor by providing detectors in two or more directions at each detection point for three-dimensional detection.

The output from the bending state detection sensor 40 is, for example, a change of the light amount based on light loss corresponding to the bending angle at each detection point. The light detected by the detectors is converted to an electric signal, and this electric signal is transmitted to the insertion assist information calculating unit 231. A table showing the relation between the bending angle and the light amount change is held, for example, in the storage unit of the insertion assist information setting unit 232 as necessary information. The number of the detectors constituting the optical fiber sensor, the location of each detector, the position relation between the detection directions indicated by an X-axis and a Y-axis and the insertion portion 12 are also held in the storage unit of the insertion assist information setting unit 232 as necessary information. The above held information is appropriately transmitted at the request of the insertion assist information calculating unit 231.

The insertion assist information calculating unit 231 calculates coordinates (X, Y, Z) of the distal end of the insertion portion 12 in three-dimensional space on the basis of the information from the insertion assist information setting unit 232 and the output from the bending state detection sensor 40. The origin of the coordinates is located, for example, on the side of the insertion portion 12 in the vicinity of the connection portion (i.e., the movable portion 13) between the grasp portion 11 and the insertion portion 12. In the present embodiment, the insertion portion 12 is made difficult to twist. That is, a twist amount relative to twisting force around the longitudinal direction in the vicinity of the grasp portion 11 in the insertion portion 12 and in the vicinity of the distal end of the insertion portion 12 is configured to be sufficiently smaller than a relative rotation amount of the insertion portion 12 and the grasp portion 11 in the movable portion 13. Thus, if the position of the proximal end of the insertion portion 12 is fixed to a certain coordinate system, the direction in which the distal end of the insertion portion 12 is viewing can be found by calculation from the information regarding the bending shape of the insertion portion 12.

As described above, the bending state detection sensor 40 outputs, as the basic information, an electric signal corresponding to the bending amount of each detector, and the insertion assist information calculating unit 231 properly combines the information from the bending state detection sensor 40, the information from the insertion assist information setting unit 232, and the information from the other sensors to calculate insertion assist information.

The rotation amount detection sensor 50 detects a rotation amount of the insertion portion 12 relative to the grasp portion 11. In the present embodiment, a rotary encoder having the circular cylindrical optical scale 125 is used as the rotation amount detection sensor 50. The sensor head 112 of the rotary encoder has a light source unit which applies light to the optical scale 125, a light receiving unit which outputs an electric signal corresponding to the light emitted from the light source unit and, for example, reflected by the optical scale 125, and a processing unit which outputs the electric signal from the light receiving unit as an electric pulse corresponding to the light-dark patterns of the scale. It is possible to calculate a rotation amount and a rotation angle of the insertion portion 12 relative to the grasp portion 11 from the number of the light-dark patterns formed in one round of the optical scale 125 provided in the scope extension 124 and from the number of electric pulses output from the sensor head 112. The number of the light-dark patterns formed in one round of the optical scale 125 is held in the storage unit of the insertion assist information setting unit 232 as necessary information. The insertion assist information calculating unit 231 calculates a relative rotation amount of the grasp portion 11 and the insertion portion 12 from the information regarding the number of the light-dark patterns and the number of electric pulses output by the sensor head 112. The output signal of the encoder is generally an analog signal having a quasi sine wave shape, and is converted to a pulse signal by an unshown signal processing circuit provided at the subsequent stage. In this instance, it is possible to set so that one pulse is output for one period of the analog signal, or it is possible to interpolate the analog signal and output more than one pulse signal. Some high-performance optical encoders are capable of outputting several thousand pulse signals for one period of the analog signal. It is possible to enhance the angular resolution by using the interpolation technique in this way. Information as to whether to interpolate and the number of interpolations is held in the storage unit of the insertion assist information setting unit 232 as necessary information, and is transmitted at the request of the insertion assist information calculating unit 231.

As described above, the rotation amount detection sensor 50 outputs an electric pulse corresponding to the rotation amount as the basic information, and the insertion assist information calculating unit 231 properly combines the information from the rotation amount detection sensor 50, the information from the insertion assist information setting unit 232, and the information from the other sensors to calculate insertion assist information.

Now, the insertion assist information is further described. As described above, the insertion assist information calculating unit 231 calculates the insertion assist information on the basis of the basic information output from the various sensors and stored in the storage unit of the insertion assist information calculating unit 231 and the necessary information from the insertion assist information setting unit 232. The insertion assist information calculating unit 231 in the present embodiment calculates, as the insertion assist information, (1) the coordinates (position) of the distal end of the insertion portion 12, the direction of the distal end, and the observation direction, relative to the grasp portion 11; (2) the coordinates (position) of the distal end of the insertion portion 12, the direction of the distal end, and the observation direction, relative to the observation target; and (3) the coordinates (position) and direction of the grasp portion 11 relative to the observation target. The information of (1) to (3) is described below.

(1) Calculation of the position, direction, and observation direction of the distal end of the insertion portion 12, relative to the grasp portion 11

As described above, the rotation direction and rotation angle of the insertion portion 12 relative to the grasp portion 11 are detected by the rotation amount detection sensor 50. In accordance with the output information from the rotation amount detection sensor 50, the direction and degree of the rotation of the insertion portion 12 relative to the grasp portion 11 can be calculated on the basis of the information from the insertion assist information setting unit 232. The three-dimensional coordinates of the insertion portion 12 relative to the proximal end of the movable portion 13 on the side of the insertion portion 12 is obtained from the output information from the bending state detection sensor 40. Therefore, it is possible to know the direction and degree of rotation of the insertion portion 12 relative to the grasp portion 11, and the position and direction of the distal end of the insertion portion 12 relative to the proximal end, so that by combining these coordinate systems, it is possible to calculate the position and direction of the distal end of the insertion portion 12 relative to the grasp portion 11. For example, the insertion portion 12 rotates $\theta$ deg relative to the grasp portion 11 around a Z-axis (the same as the Z-axis in FIG. 4), and the coordinates of the distal end of the insertion portion 12 are (x1, y1, z1) relative to the proximal end of the insertion portion 12, in which case the position (x2, y2, z2) of the distal end of the insertion portion 12 relative to the grasp portion 11 is represented by the following expression.

$$\begin{pmatrix} x2 \\ y2 \\ z2 \end{pmatrix} = R^{-1} \begin{pmatrix} x1 \\ y1 \\ z1 \end{pmatrix} = \begin{pmatrix} \cos\theta & \sin\theta & 0 \\ -\sin\theta & \cos\theta & 0 \\ 0 & 0 & 1 \end{pmatrix} \begin{pmatrix} x1 \\ y1 \\ z1 \end{pmatrix} = \begin{pmatrix} x1\cos\theta + y1\sin\theta \\ -x1\sin\theta + y1\cos\theta \\ z1 \end{pmatrix}$$

[Expression 1]

wherein $R^{-1}$ in (Expression 1) is an inverse rotation matrix. The direction and observation direction of the distal end can also be easily converted to coordinates by performing a similar calculation in the form of a vector. Thus, the calculation in (Expression 1) is performed by properly associating (combining) the output information from the bending state detection sensor 40 with the output information from the rotation amount detection sensor 50.

(2) Calculation of the position, direction, and observation direction of the distal end of the insertion portion 12, relative to the observation target

For the position of the distal end of the insertion portion 12 relative to the observation target, coordinate conversion of the observation target has only to be performed by use of the information from the insertion amount detection sensor 30 in addition to the calculation shown in (1). The position relation between the observation target and the insertion portion 12 is detected by the insertion amount detection sensor 30. When a certain position of the insertion portion 12 is located at the position of the insertion amount detection sensor 30, this position is determined as an origin to calculate the coordinate position of the distal end of the insertion portion 12, so that the position of the distal end of the insertion portion 12 relative to the observation target can be calculated. When the coordinates of the origin relative to the grasp portion 11 are (x3, y3, z3), the position (x4, y4, z4) of the distal end of the insertion portion 12 relative to the observation target is (x2-x3, y2-y3, z2-z3). The direction and observation direction of the distal end can also be easily converted to coordinates by performing a similar calculation in the form of a vector. Thus, the calculation of (2) is performed by further associating (combining) the output information from the insertion amount detection sensor 30.

(3) Calculation of the position and direction of the grasp portion 11 relative to the observation target

In the present embodiment, the bending state detection sensor 40 is provided over the whole insertion portion 12. Thus, when a certain position of the insertion portion 12 is located at the position of the insertion amount detection sensor 30, the position of the grasp portion 11 relative to the insertion amount detection sensor 30 can be found from the shape detection result of the part on the side of the grasp portion 11 by the bending state detection sensor 40 and from the detection result by the rotation amount detection sensor 50. That is, a calculation similar to the technique shown in (2) has only to be performed not for the distal end of the insertion portion 12 but for the grasp portion 11. The direction of the grasp portion 11 and the direction in which the grasp portion 11 is located can also be easily converted to coordinates by performing a similar calculation in the form of a vector.

Necessary information among the insertion assist information calculated by the procedures in (1) to (3) is properly selected by the insertion assist information selecting unit 233, and the selected insertion assist information is provided to the operator by the output means.

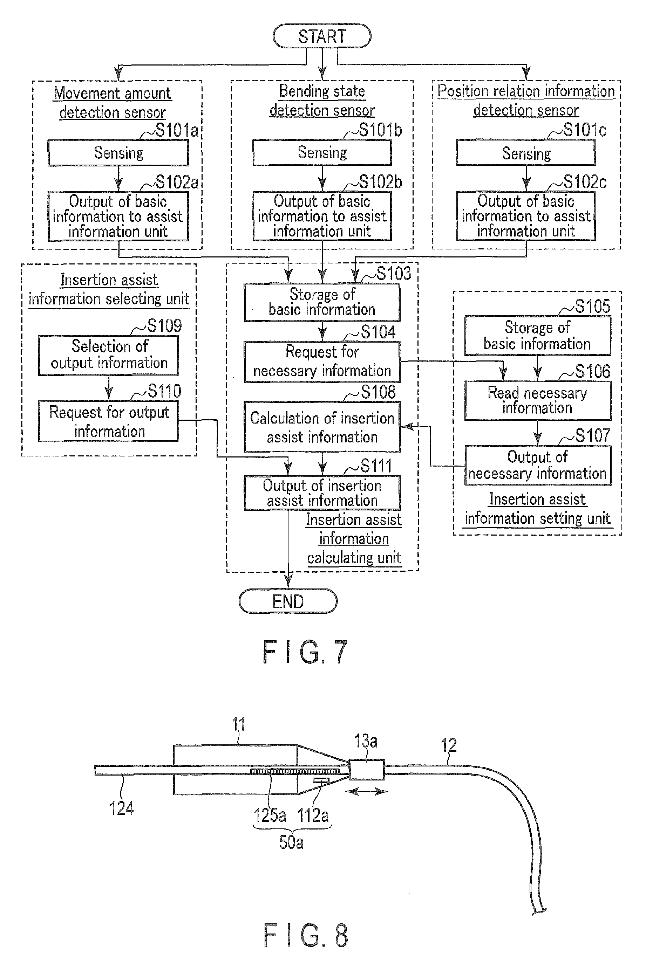

An example of a flowchart showing the flow of processing from the detection of the basic information by the various sensors to the output of the insertion assist information is shown in FIG. 7. If the processing in FIG. 7 is started, the layout information detection sensor (insertion amount detection sensor 30), the bending state detection sensor (bending state detection sensor 40), and the movement amount detection sensor (rotation amount detection sensor 50) respectively detect the basic information in accordance with the above-mentioned detection techniques (S101a, S101b, and S101c) . When the detection of the basic information is finished, the insertion amount detection sensor 30, the bending state detection sensor 40, and the rotation amount detection sensor 50 output the acquired basic information to the insertion assist information calculating unit 231 (S102a, S102b, and S102c).

The insertion assist information calculating unit 231 temporarily stores the basic information input from the three sensors in the storage unit (S103). The insertion assist information calculating unit 231 then requests the insertion assist information setting unit 232 for necessary information which is necessary for the calculation of insertion assist information, in accordance with the kind of basic information stored in the storage unit (S104).

The insertion assist information setting unit 232 has previously stored the necessary information in the storage unit (S105). At the request of the insertion assist information calculating unit 231, the insertion assist information setting unit 232 reads the requested necessary information from the storage unit (S106). The insertion assist information setting unit 232 then outputs the read necessary information to the insertion assist information calculating unit 231 (S107).

The insertion assist information calculating unit 231 uses the basic information acquired from the various sensors and the necessary information acquired from the insertion assist information setting unit 232 to calculate insertion assist information (S108).

For example, in accordance with a program input from the outside or prestored in the storage unit, the insertion assist information selecting unit 233 selects the insertion assist information output by use of the output means, among the insertion assist information calculated by the insertion assist information calculating unit 231 (S109). The insertion assist information calculating unit 231 then requests the output of the selected insertion assist information from the insertion assist information calculating unit 231 (S110).

At the request of the insertion assist information selecting unit 233, the insertion assist information calculating unit 231 reads the selected insertion assist information from the storage unit, and outputs the read insertion assist information to the predetermined output means (S111). In this instance, the output means displays the insertion assist information from the insertion assist information calculating unit 231 on a display unit so that the insertion assist information is available to, for example, the operator.

Here, the flow of the processing shown in FIG. 7 is only one example. For example, the order of some of the processing can be changed with regard to time. It is also possible to omit some of the processing, or perform the processing in parallel with other processing. Processing that is not shown in FIG. 7 can be performed at various timings without departing from the spirit of the present invention.

Although the calculations shown in (1) to (3) perform coordinate conversion to adjust the information detected in accordance with the coordinate system of each of the various sensors to the coordinate system of the necessary insertion assist information, this is not a limitation. Various existing algorithms can be used to calculate insertion assist information; for example, a coordinate system common to all the sensors is set in advance, and insertion assist information is calculated on the basis of this coordinate system.

As has been described above, according to the first embodiment, even in the case of the scope 10 having the movable portion 13 which drives the insertion portion 12 independently of the grasp portion 11, it is possible to inform the operator of insertion assist information such as the position and direction of the distal end of the insertion portion 12, and observation direction. By informing the operator of the insertion assist information in this way, it is possible to significantly improve convenience during the insertion of the insertion portion 12 and during observation operation. It is also possible to reduce erroneous operations and observation errors.

Although the movable portion 13 has a structure that can rotate the insertion portion 12 relative to the grasp portion 11 in the example described according to the first embodiment, this is not a limitation. That is, the technique according to the present embodiment is applicable to the movable portion 13 having a structure that drives the insertion portion 12 relative to the grasp portion 11 independently of its bending operation. For example, as shown in FIG. 8, a movable portion 13a may have a structure that drives the insertion portion 12 to be translated relative to the grasp portion 11. In the case of the structure in FIG. 8, a movement amount detection sensor 50a is used as a movement amount detection sensor instead of the rotation amount detection sensor 50 used in the present embodiment. The movement amount detection sensor 50a is, for example, an optical or magnetic linear encoder. In the case of the linear encoder, a sensor head 112a and a scale 125a translate relative to each other. In the case of a movable portion capable of both rotational movement and translational movement, the rotation amount detection sensor and the movement amount detection sensor are used in combination.

Although the rotation angle of the insertion portion 12 relative to the grasp portion 11 is not limited in the example shown in the present embodiment, the rotation angle of the insertion portion 12 can be limited to a practical angular range. For example, an angular range smaller than 360 degrees is sufficient to only view in necessary directions. By limiting the rotation angle of the insertion portion 12 in this way, it is possible to reduce an area where a scale as the rotation amount detection sensor 50 needs to be provided. It is also possible to prevent, for example, unshown wiring lines in the insertion portion 12 from being twisted and thus broken.

Although the example of application to a medical endoscope apparatus for observing a living body is mainly assumed and described in the first embodiment, this is not a limitation. The technique according to the present embodiment is also applicable to industrial endoscope apparatuses for observing engines of aircrafts and automobiles or plant pipes. Even in the case of the industrial endoscope apparatuses, the layout relation between the insertion portion and the grasp portion is detected, and the information is conveyed to the operator by, for example, display, so that the insertion operation and observation operation of the insertion portion by the operator are more easily performed.

<Second Embodiment>

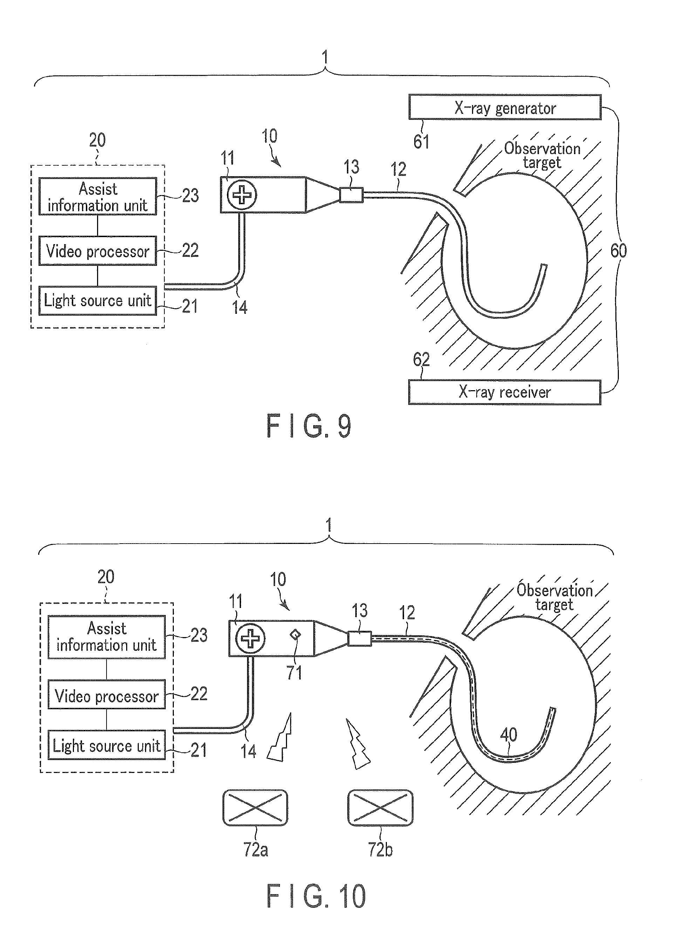

The second embodiment of the present invention is now described with reference to FIG. 9. FIG. 9 is a block diagram showing a configuration that uses an X-ray imaging apparatus. The same parts in the second embodiment as those in the first embodiment are not described, and differences are only described. The optical fiber sensor is used as the bending state detection sensor 40 in the example shown in the first embodiment. In contrast, an X-ray imaging technique is used in the example described in the second embodiment. According to the X-ray imaging technique, the X-ray imaging apparatus having an X-ray generator and an X-ray receiver across an observation target is disposed, and X-rays are applied to the observation target from the X-ray generator so that the X-rays will transmit the observation target, and the X-rays are detected by the X-ray receiver. The insertion portion 12 in the endoscope apparatus has the properties of not easily transmitting X-rays compared to living cells and the like. Therefore, the entire shape, for example, the bending shape of the insertion portion 12 can be detected by use of the X-ray imaging technique.

The configuration of an insertion assist information detection system of the endoscope apparatus according to the second embodiment is substantially equal to the configuration shown in FIG. 1, and is different in that the bending state detection sensor 40 is not mounted in the insertion portion 12. Instead, in the present embodiment, an X-ray imaging apparatus 60 having an X-ray generator 61 and an X-ray receiver 62 across an observation target is disposed, as shown in FIG. 9.

In the shape detection of the endoscope apparatus according to the X-ray imaging technique, a projection image of the insertion portion 12 is detected by the X-ray receiver 62. Thus, the bending shape of the insertion portion 12 is a two-dimensional shape which is a projection on a surface including the receiving surface of the X-ray receiver 62. A plane on which such a two-dimensional image is projected is generally, for example, a bed on which a human body that is the observation target lies.

Conversion information for a distance to unify the coordinate systems of the two-dimensional detection information for the insertion portion 12 and the rotation amount detection sensor 50 provided in the vicinity of the movable portion 13 is previously stored in the storage unit of the insertion assist information setting unit 232. The insertion assist information calculating unit 231 uses this conversion information for the distance to calculate insertion assist information such as the position and direction of the distal end of the insertion portion 12, and observation direction. That is, the insertion assist information calculating unit 231 properly combines the position of the distal end of the insertion portion 12 on an X-ray image, the position of the grasp portion 11, the output of the rotation amount detection sensor 50, and output information from the insertion amount detection sensor 30 to calculate insertion assist information. It is also possible to use two pairs of X-ray imaging apparatuses 60, and detect the bending shape of the insertion portion 12 from different directions to acquire three-dimensional information regarding the insertion portion 12. Otherwise, it is also possible to configure a pair of X-ray imaging apparatuses 60 rotatably around the observation target to acquire three-dimensional information regarding the insertion portion 12.

Thus, in the present embodiment, it is possible to detect various kinds of insertion assist information by using the X-ray imaging apparatus 60 without mounting any sensor in the insertion portion 12.

Although the X-ray imaging technique is used to detect the bending shape of the insertion portion 12 in the example shown here in the present embodiment, this is not a limitation. For example, it is possible to use a magnetic sensor technique wherein magnetic coils are mounted in the insertion portion 12 and the positions and directions of the magnetic coils are detected by an externally provided antenna. Such a configuration permits the bending shape of the insertion portion 12 to be detected without exposing the observation target to X-rays.

<Third Embodiment>

Now, the third embodiment of the present invention is described with reference to FIG. 10. The same parts in the third embodiment as those in the first embodiment are not described, and differences are only described. The insertion amount detection sensor 30 is used as the layout relation detection sensor in the example shown in the first embodiment. In contrast, a position sensor mounted in the grasp portion 11 is used as the position relation detection sensor in the third embodiment.

As shown in FIG. 10, the position sensor as the position relation detection sensor according to the present embodiment has an electric wave emitter 71 mounted in the grasp portion 11, and antennas 72a and 72b arranged in an observation room. The antenna 72a and the antenna 72b are arranged with a predetermined space, and are connected to an unshown position detection circuit.

Electric waves released from the electric wave emitter 71 propagate in the space inside the observation room, and reach each of the antennas 72a and 72b properly arranged in the observation room. The unshown position detection circuit specifies the position and direction of the electric wave emitter 71 from the difference between times at which the electric waves have reached the antenna 72a and the antenna 72b, and transmits position information and direction information to the insertion assist information calculating unit 231. The insertion assist information calculating unit 231 calculates insertion assist information from the position information and the direction information regarding the electric wave emitter 71.

As described above, the position sensor as the position relation detection sensor according to the third embodiment outputs an electric signal corresponding to the position of the grasp portion 11 as the basic information. The insertion assist information calculating unit 231 properly combines the electric signal corresponding to the position and direction of the grasp portion 11, the information from the insertion assist information setting unit 232, and the information from the other sensors to calculate insertion assist information.

Here, in the first embodiment, the position, direction, and observation direction of the distal end of the insertion portion 12 relative to the grasp portion 11 or the observation target is calculated as insertion assist information from the output information from the insertion amount detection sensor 30, the output information from the bending state detection sensor 40, and the output information from the rotation amount detection sensor 50. In contrast, in the present embodiment, the output information from the position sensor, the output information from the bending state detection sensor 40, and the output information from the rotation amount detection sensor 50 are combined. Since the insertion amount detection sensor 30 is not present, the position and direction of the grasp portion 11 are used as references, and a change of the bending state of the insertion portion 12 is detected with respect to the above position, so that the position and direction of the distal end of the insertion portion 12, and observation direction relative to the grasp portion 11 can be calculated as in the first embodiment. Moreover, it is not necessary to dispose a sensor in the vicinity of the opening of the observation target in the third embodiment. Therefore, it is possible to detect insertion assist information without impairing the workability of the operator.

Although the electric wave emitter 71 is mounted in the grasp portion 11 alone in the example shown here in the present embodiment, this is not a limitation. For example, by attaching the electric wave emitter 71 to the observation target as well, it is possible to detect the layout relation between the observation target and the grasp portion 11. Thus, it is possible to provide various kinds of insertion assist information regarding the observation target similar to that in the first embodiment.

Although the electric wave emitter and the antennas are combined as the position sensor in the example shown in the present embodiment, this is not a limitation. Various modifications can be made, such as a combination of a sound wave emitter and a microphone, a combination of a visible ray emitter and a receiver, a combination of an infrared emitter and a receiver, and a combination of a magnetic emitter and a magnetic antenna. By properly combining these components, it is possible to improve detection accuracy or widen the range of application to various environments and observation targets.

Although the electric wave emitter 71 is disposed in the grasp portion 11 and the antennas 72a and 72b are disposed outside the grasp portion 11 in the example in FIG. 10, this is not a limitation. Conversely, an antenna may be disposed in the grasp portion 11, and electric wave emitters may be disposed outside.

Furthermore, an acceleration sensor may be mounted in the grasp portion 11 instead of disposing the electric wave emitter and the antennas, and the position of the grasp portion 11 may be detected by converting an acceleration change to positional information. A general conventional technique can be used as a position detection method that uses the acceleration sensor. That is, acceleration information can be converted to positional information by integrating the acceleration information two times.

While the present invention has been described above in connection with the embodiments, the present invention is not limited to the embodiments described above. For example, in all the embodiments described above, the insertion portion 12 is bendable. The insertion portion 12 may be unbendable. That is, the technique according to the present embodiment is applicable to not only a flexible endoscope but also a rigid endoscope. Here, when the insertion portion 12 is configured to be unbendable, the bending state detection sensor 40 is not necessary. Therefore, insertion assist information is calculated on the basis of the basic information from the movement amount detection sensor and the position relation detection sensor and the information from the insertion assist information setting unit 232. A designer or a user of the apparatus may properly select insertion assist information suited to an endoscope apparatus using the hard endoscope and its system to be applied among the insertion assist information described in the above embodiments.

The present application includes the following inventions in addition to the invention described in the claims.

[1] The insertion assist information detection system of the endoscope apparatus according to claim 4, wherein the scale is an optical scale having a periodic optical pattern, and
the sensor head applies light to the optical scale, and receives the light which has been applied and gone through the scale and then outputs an electric signal.
[2] The insertion assist information detection system of the endoscope apparatus according to claim 4, wherein the scale is a magnetic scale having a periodic magnetic pattern, and
the sensor head detects a change of a magnetic field resulting from the movement of the magnetic scale and then outputs an electric signal.
[3] The insertion assist information detection system of the endoscope apparatus according to claim 8, wherein twist amounts, around a longitudinal direction, of a part of the insertion portion in the vicinity of the grasp portion and a part in the vicinity of the distal end of the insertion portion are sufficiently smaller than a relative rotation amount in the movable portion.
[4] The insertion assist information detection system of the endoscope apparatus according to claim 22, wherein the movable portion mechanically connects the insertion portion and the grasp portion to rotate the insertion portion relative to the grasp portion,

the rotation axis of the rotation is provided to be located in a direction substantially corresponding to the longitudinal direction of the insertion portion and in a region inside the insertion portion, and

the movement amount detection sensor is a rotation amount detection sensor which detects a relative rotation amount of the insertion portion and the grasp portion.

[5] The endoscope apparatus according to claim 23, wherein the insertion portion is configured to at least partly bend independently of the relative movement by the movable portion,

the endoscope apparatus further comprising

a bending state detection sensor which detects the bending state of the insertion portion, and

an insertion assist information calculating unit which associates a movement amount detected by the movement amount detection sensor with the bending state detected by the bending state detection sensor to calculate insertion assist information.

[6] The endoscope apparatus according to claim 24, wherein the bending state detection sensor is an optical fiber sensor comprising, in a part of the longitudinal direction of an optical fiber mounted in the insertion portion, at least one detector which detects a change of at least one of the amount, wavelength, intensity, and phase of light guided by the optical fiber in response to the bending angle of the optical fiber.

[7] The endoscope apparatus according to claim 23, further comprising a position relation detection sensor which detects a relative position relation between the insertion portion and an observation target having the tube.

**Claims**

1. An insertion assist information detection system for an endoscope apparatus, the system comprising:

   the endoscope apparatus comprising:

   an insertion portion to be inserted into a tube,
   a grasp portion which is grasped by an operator, and
   a movable portion which mechanically connects the insertion portion and the grasp portion to relatively move the insertion portion and the grasp portion; and
   a movement amount detection sensor which detects a relative movement amount of the insertion portion and the grasp portion in the movable portion.

2. The insertion assist information detection system for the endoscope apparatus according to claim 1, wherein the movable portion mechanically connects the insertion portion and the grasp portion to rotate the insertion portion relative to the grasp portion,

   the rotation axis of the rotation is provided to be located in a direction substantially corresponding to the longitudinal direction of the insertion portion and in a region inside the insertion portion, and

   the movement amount detection sensor is a rotation amount detection sensor which detects a relative rotation amount of the insertion portion and the grasp portion.

3. The insertion assist information detection system for the endoscope apparatus according to claim 2, wherein the insertion portion has a substantially circular cylindrical shape or substantially circular cylindrical member in the vicinity of the grasp portion, and the rotation axis substantially corresponds to a center axis of the circular cylinder.

4. The insertion assist information detection system for the endoscope apparatus according to claim 3, wherein the rotation amount detection sensor includes a scale, and a sensor head which detects the movement of the scale, and one of the scale and the sensor head is provided in the insertion portion, and the other is provided in the grasp portion.

5. The insertion assist information detection system for the endoscope apparatus according to claim 2, wherein the insertion portion is configured to at least partly bend independently of the relative movement by the movable portion, the insertion assist information detection system further comprising:

   a bending state detection sensor which detects a bending state of the insertion portion, and
   an insertion assist information calculating unit which associates a movement amount detected by the movement amount detection sensor with the bending state detected by the bending state detection sensor to calculate insertion assist information.

6. The insertion assist information detection system for the endoscope apparatus according to claim 5, wherein the bending state detection sensor detects substantially an entire shape of the insertion portion, and the insertion assist information calculating unit combines substantially the entire shape of the insertion portion detected by the bending state detection sensor with the movement amount detected by the movement amount detection sensor to calculate, as the insertion assist information, at least one of the position of the distal end of the insertion portion relative to the grasp portion, the direction of the distal end of the insertion portion relative to the grasp portion, and the observation direction of the insertion portion relative to the grasp portion.

7. The insertion assist information detection system for the endoscope apparatus according to claim 6, wherein a relative rotation amount of the insertion portion and the grasp portion in the movable portion is smaller than 360 degrees.

8. The insertion assist information detection system for the endoscope apparatus according to claim 1, further comprising a position relation detection sensor which detects a relative position relation between the insertion portion and an observation target having the tube.

9. The insertion assist information detection system for the endoscope apparatus according to claim 8, wherein the position relation detection sensor is an insertion amount detection sensor which is attached to the observation target and which detects, as an insertion state of the insertion portion, at least one of an insertion amount, a rotation amount, and an insertion angle of the insertion portion relative to the observation target.

10. The insertion assist information detection system for the endoscope apparatus according to claim 9, wherein the insertion portion is configured to at least partly bend independently of the relative movement by the movable portion, the insertion assist information detection system further comprising:

   a bending state detection sensor which detects a bending state of the insertion portion, and
   an insertion assist information calculating unit which associates a movement amount detected by the movement amount detection sensor, the bending state detected by the bending state detection sensor, and the insertion state detected by the insertion amount detection sensor with one another to calculate, as insertion assist information regarding the endoscope apparatus, information regarding at least one of the position of the distal end of the insertion portion relative to the observation target, the direction of the distal end of the insertion portion relative to the observation target, and the observation direction of the insertion portion relative to the observation target.

11. The insertion assist information detection system for the endoscope apparatus according to claim 9, wherein the insertion portion is configured to at least partly bend independently of the relative movement by the movable portion, the insertion assist information detection system further comprising a bending state detection sensor which detects a bending state of the insertion portion, and a movement amount detected by the movement amount detection sensor being associated with the bending state detected by the bending state detection sensor to calculate, as insertion assist information regarding the endoscope apparatus, the position of the grasp portion relative to the observation target.

12. The insertion assist information detection system for the endoscope apparatus according to claim 8, wherein the position relation detection sensor is a position sensor which detects the positions of the grasp portion and/or the observation target.

13. The insertion assist information detection system for the endoscope apparatus according to claim 12, wherein the position sensor includes an acceleration sensor, and a relative position relation between the insertion portion and the observation target is calculated as a movement direction and a movement amount of the grasp portion relative to a condition in which the position sensor and the observation target are disposed at predetermined positions.

14. The insertion assist information detection system for the endoscope apparatus according to claim 12, wherein the position sensor includes an emitter which emits a signal and an antenna which receives the signal, and one of the antenna and the emitter is attached to the grasp portion, and the other is disposed in the observation target or in an observation room where an observation operation of the observation target is performed.

15. The insertion assist information detection system for the endoscope apparatus according to claim 14, wherein the signal emitted by the emitter is one or a combination of electric waves, a magnetic signal, visible rays, infrared rays, and a sound wave signal.

16. The insertion assist information detection system for the endoscope apparatus according to claim 5, wherein the bending state detection sensor is mounted in the insertion portion.

17. The insertion assist information detection system of the endoscope apparatus according to claim 16, wherein the bending state detection sensor is an optical fiber sensor comprising, in a part of the longitudinal direction of an optical fiber mounted in the insertion portion, at least one detector which detects a change of at least one of the amount, wavelength, intensity, and phase of light guided by the optical fiber in response to the bending angle of the optical fiber.

18. The insertion assist information detection system of the endoscope apparatus according to claim 5, wherein the bending state detection sensor is an X-ray imaging apparatus comprising an X-ray generator and an X-ray receiver outside the insertion portion.

19. An endoscope apparatus comprising:

an insertion portion to be inserted into a tube;
a grasp portion which is grasped by an operator;
a movable portion which mechanically connects the insertion portion and the grasp portion to relatively move the insertion portion and the grasp portion; and
a movement amount detection sensor which detects a relative movement amount of the insertion portion and the grasp portion in the movable portion.

FIG. 1

FIG. 2

FIG. 3

FIG. 4

FIG. 5

Assist information unit ⌐-23

Insertion assist information setting unit ⌐232

⇩

Insertion assist information calculating unit ⌐231

⇧

Insertion assist information selecting unit ⌐233

Insertion assist information

Output means

50

Rotation amount detection sensor (optical encoder)

40

Bending state detection sensor (optical fiber sensor)

30

Insertion amount detection sensor (speckle sensor)

FIG. 6

START

Movement amount
detection sensor
~S101a
Sensing
~S102a
Output of basic
information to assist
information unit

Bending state
detection sensor
~S101b
Sensing
~S102b
Output of basic
information to assist
information unit

Position relation information
detection sensor
~S101c
Sensing
~S102c
Output of basic
information to assist
information unit

Insertion assist
information selecting unit
~S109
Selection of
output information
~S110
Request for output
information

~S103
Storage of
basic information
~S104
Request for
necessary information
~S108
Calculation of insertion
assist information
~S111
Output of insertion
assist information
Insertion assist
information
calculating unit

~S105
Storage of
basic information
~S106
Read necessary
information
~S107
Output of
necessary information
Insertion assist
information setting unit

END

F I G. 7

F I G. 8

11
13a
12
124
125a  112a
50a

F I G. 9

F I G. 10

## INTERNATIONAL SEARCH REPORT

International application No.

PCT/JP2013/081099

| A. CLASSIFICATION OF SUBJECT MATTER |
| --- |
| *A61B1/00*(2006.01)i, *G02B23/24*(2006.01)i |

According to International Patent Classification (IPC) or to both national classification and IPC

| B. FIELDS SEARCHED |
| --- |
| Minimum documentation searched (classification system followed by classification symbols) |
| A61B1/00, G02B23/24 |

Documentation searched other than minimum documentation to the extent that such documents are included in the fields searched

| | | | |
| --- | --- | --- | --- |
| Jitsuyo Shinan Koho | 1922–1996 | Jitsuyo Shinan Toroku Koho | 1996–2014 |
| Kokai Jitsuyo Shinan Koho | 1971–2014 | Toroku Jitsuyo Shinan Koho | 1994–2014 |

Electronic data base consulted during the international search (name of data base and, where practicable, search terms used)

| C. DOCUMENTS CONSIDERED TO BE RELEVANT | | |
| --- | --- | --- |
| Category* | Citation of document, with indication, where appropriate, of the relevant passages | Relevant to claim No. |
| X<br>Y | JP 61-122618 A  (Olympus Optical Co., Ltd.),<br>10 June 1986 (10.06.1986),<br>page 2, lower left column, line 13 to page 3,<br>lower right column, line 20; all drawings<br>(Family: none) | 1,19<br>2-18 |
| Y | JP 2005-254002 A  (Olympus Corp.),<br>22 September 2005 (22.09.2005),<br>abstract<br>(Family: none) | 2-7,16-18 |
| Y | JP 2010-234058 A  (Olympus Medical Systems<br>Corp.),<br>21 October 2010 (21.10.2010),<br>abstract<br>(Family: none) | 2-7,16-18 |

☒ Further documents are listed in the continuation of Box C.　　　☐ See patent family annex.

| | |
| --- | --- |
| *　Special categories of cited documents:<br>"A"　document defining the general state of the art which is not considered　to be of particular relevance<br>"E"　earlier application or patent but published on or after the international filing date<br>"L"　document which may throw doubts on priority claim(s) or which is cited to establish the publication date of another citation or other special reason (as specified)<br>"O"　document referring to an oral disclosure, use, exhibition or other means<br>"P"　document published prior to the international filing date but later than the priority date claimed | "T"　later document published after the international filing date or priority date and not in conflict with the application but cited to understand the principle or theory underlying the invention<br>"X"　document of particular relevance; the claimed invention cannot be considered novel or cannot be considered to involve an inventive step when the document is taken alone<br>"Y"　document of particular relevance; the claimed invention cannot be considered to involve an inventive step when the document is combined with one or more other such documents, such combination being obvious to a person skilled in the art<br>"&"　document member of the same patent family |

| | |
| --- | --- |
| Date of the actual completion of the international search<br>　18 February, 2014 (18.02.14) | Date of mailing of the international search report<br>　04 March, 2014 (04.03.14) |
| Name and mailing address of the ISA/<br>　Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2013/081099

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2003-225195 A  (Pentax Corp.),<br>12 August 2003 (12.08.2003),<br>paragraphs [0009] to [0078]; all drawings<br>(Family: none) | 5-11,16,17 |
| Y | JP 2011-30735 A  (Fujifilm Corp.),<br>17 February 2011 (17.02.2011),<br>abstract<br>(Family: none) | 5-7,16,17 |
| Y | JP 56-139733 A  (Olympus Optical Co., Ltd.),<br>31 October 1981 (31.10.1981),<br>claims; all drawings<br>(Family: none) | 5-7,18 |
| Y | JP 59-7919 A  (Olympus Optical Co., Ltd.),<br>17 January 1984 (17.01.1984),<br>page 2, lower right column, line 15 to page 4,<br>lower left column, line 6; all drawings<br>(Family: none) | 8-11 |
| Y | JP 2012-70937 A  (Fujifilm Corp.),<br>12 April 2012 (12.04.2012),<br>abstract<br>(Family: none) | 8-11 |
| Y | JP 2012-235983 A  (Olympus Medical Systems<br>Corp.),<br>06 December 2012 (06.12.2012),<br>abstract; paragraph [0022]<br>(Family: none) | 8-15 |
| Y | WO 2010/050526 A1  (Olympus Medical Systems<br>Corp.),<br>06 May 2010 (06.05.2010),<br>entire text; all drawings<br>& JP 4759654 B        & US 2011/0098533 A1<br>& EP 2351509 A1        & CN 102196761 A | 5-16,17 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005254002 A